# FASCICULE DE BREVET EUROPEEN

(11) **EP 3 538 882 B1**
(45) Date de publication et mention de la délivrance du brevet: **19.08.2020**
(21) Numéro de dépôt: 17801397.5
(22) Date de dépôt: 09.11.2017
(51) Int. Cl.: G01N 27/413, B01L 3/00

(54) **DISPOSITIF, SYSTÈME ET PROCÉDÉ RELATIF À LA PRÉCONCENTRATION D'ANALYTES**
VORRICHTUNG, SYSTEM UND VERFAHREN IM ZUSAMMENHANG MIT DER VORKONZENTRIERUNG VON ANALYTEN
DEVICE, SYSTEM AND METHOD RELATIVE TO THE PRECONCENTRATION OF ANALYTES

(30) Priorité: 09.11.2016 FR 1660855
(43) Date de publication de la demande: 18.09.2019
(73) Titulaire: Centre National de la Recherche Scientifique CNRS, 75016 Paris (FR)
(72) Inventeur: DELAPIERRE, François-Damien, 94230 Cachan (FR); PALLANDRE, Antoine, 92330 Sceaux (FR); GUILET, Stéphane, 91530 Saint-Cheron (FR); HAGHIRI-GOSNET, Anne-Marie, 92330 Sseaux (FR); CAMBRIL, Edmond, 92290 Chatenay Malabry (FR)
(74) Mandataire: Regimbeau
(86) Numéro de dépôt international: PCT/EP2017/078785
(87) Numéro de publication internationale: WO 2018/087240

(56) Documents cités:
- WO-A1-2010/034908
- ANNE-CLAIRE LOUËR ET AL: "Pressure-Assisted Selective Preconcentration in a Straight Nanochannel", ANALYTICAL CHEMISTRY, vol. 85, no. 16, 20 août 2013 (2013-08-20) , pages 7948-7956, XP055390382, US ISSN: 0003-2700, DOI: 10.1021/ac4016159 cité dans la demande
- Anne Claire Louer: "Préconcentration sélective immunologique en nanofluidique : vers l'identification rapide d'agents du risque biologique", , 25 octobre 2013 (2013-10-25), XP055390393, Extrait de l'Internet: URL:https://tel.archives-ouvertes.fr/tel-0 0876894/document [extrait le 2017-07-12]
- WANG ZHONGWEI ET AL: "Rapid detection and quantification of bacteria using an integrated micro/nanofluidic device", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 178, 16 janvier 2013 (2013-01-16), pages 683-688, XP028993269, ISSN: 0925-4005, DOI: 10.1016/J.SNB.2013.01.017
- SUNG HEE KO ET AL: "Nanofluidic preconcentration device in a straight microchannel using ion concentration polarization", LAB ON A CHIP, vol. 12, no. 21, 24 juillet 2012 (2012-07-24), page 4472, XP055390784, ISSN: 1473-0197, DOI: 10.1039/c2lc21238b

## Description

### DOMAINE TECHNIQUE GENERAL

L'invention concerne le domaine des dispositifs, systèmes, et procédés pour analyser et/ou discriminer et/ou trier des particules ou des molécules présentes dans une solution (on parlera plus généralement d'analytes, chargés ou non contenus dans un électrolyte).

L'invention permet également de faire des réactions chimiques et biochimiques en mélangeant de manière très locale deux groupes de molécules présentes en très faible quantité.

L'invention propose une solution dans le domaine de la microfluidique exploitant la compétition des mobilités électrophorétique, et avantageusement hydrodynamique (ajout de pression supplémentaire, flux de Poiseuille) et électroosmotique, et plus particulièrement l'électropréconcentation.

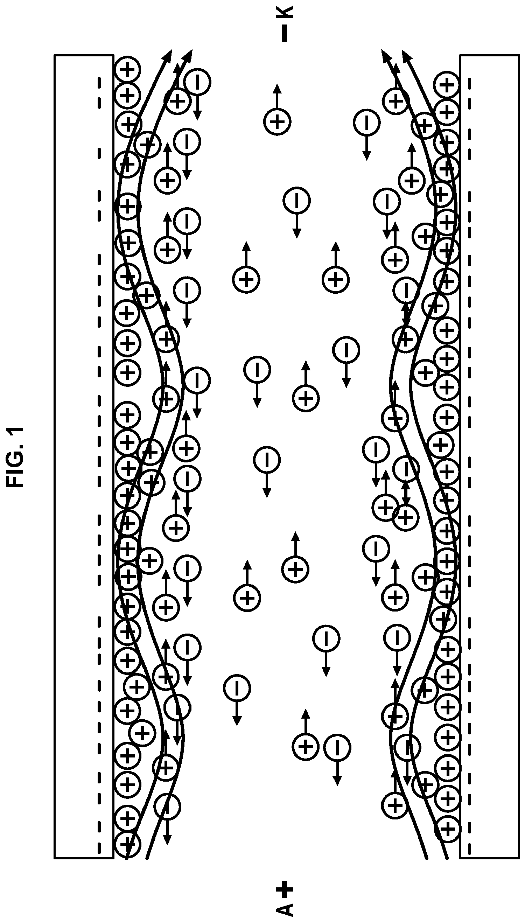

En **figure 1** est illustré un microcanal en verre rempli d'une solution ionique. Dès que le pH est supérieur à 2 environ, les parois sont négativement chargées. Par conséquent, les cations (illustrés par des « + ») présents dans l'électrolyte de fond s'accumulent au voisinage des parois. Les cations les plus proches de celles-ci ne peuvent pas se déplacer, mais les couches de cations suivantes, elles, se déplacent vers une cathode K dès qu'un champ électrique est appliqué, entrainant tout le liquide.

Il s'agit du flux électro-osmotique, qui dépend de la charge de surface du verre ou encore du potentiel zeta de surface et qui existe même en l'absence d'analyte dans l'électrolyte de fond

Dans le même temps, les anions (illustrés par des « - ») et les cations présents partout ailleurs dans le liquide migrent eux aussi, respectivement à l'anode A et à la cathode K.

On parle de flux électrophorétique, qui dépend du rapport charge/masse de l'analyte.

Dans le cas d'un microcanal à la géométrie simple, comme illustré sur la **figure 1** (pas de restriction, de membranes ou de modification locale de la section), ces deux flux sont de sens opposé (voir les flèches) pour les anions et de même sens pour les cations.

Lorsque les migrations de certains de ces ions sont perturbées, par exemple par une restriction de taille nanométrique, par une membrane poreuse, ou simplement par le recouvrement local des parois par une substance captant certains ions, des phénomènes d'accroissement local de la concentration ionique et par conséquent de raréfaction des ions en d'autres points sont observés.

On parle d'électropréconcentration.

Ce phénomène est de grand intérêt à de nombreux points de vue, il permet en effet de concentrer des molécules très diluées pour en faciliter la détection et/ou de séparer des ions, qui ne se concentreraient pas aux mêmes endroits.

### ETAT DE L'ART

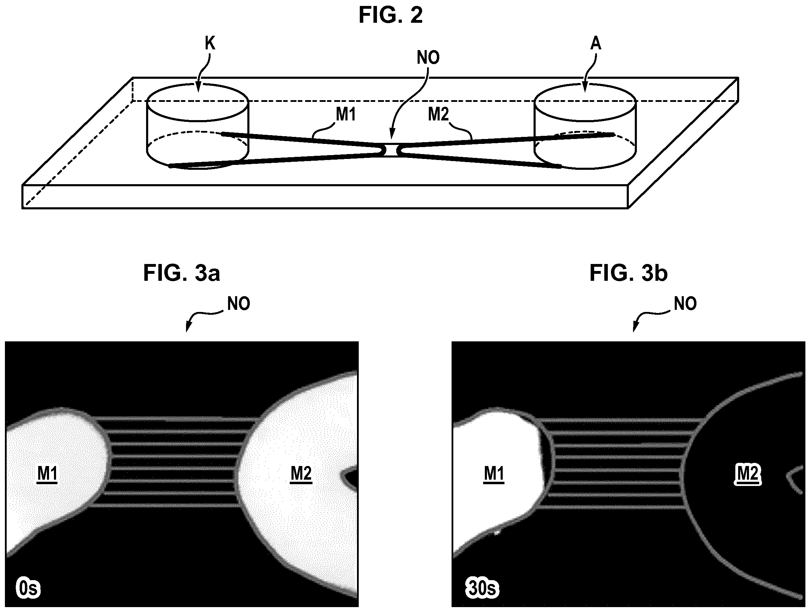

Les premières observations du phénomène de préconcentration ionique autour d'une nanofente ont été effectuées par Pu et al. en 2004 [Pu-2004] (les références sont détaillées en fin de description). Le dispositif qui a permis ces observations, schématisé en **figure 2****,** est constitué de deux microcanaux M1, M2 de 100µm de profondeur en forme de U reliés par huit nanocanaux N0 de 60 nm de profondeur au niveau de la courbure du U. Chaque nanocanal forme une restriction nanométrique qui perturbe le transfert des anions, ce qui génère le phénomène de préconcentration.

La préconcentration est alors notamment fonction de la nature et/ou de la géométrie de la restriction, et des caractéristiques biophysiques de l'analyte telles que sa mobilité.

En **figures 3a** et **3b** sont représentés les résultats avant électropréconcentration et après électropréconcentration. L'ensemble est rempli d'une solution contenant de la fluorescéine ou de la rhodamine 6G diluée dans une solution de tétraborate de sodium (**figure 3a**). Lorsque des champs électriques de 1kV sont appliqués entre l'anode A et la cathode K, les ions se concentrent du côté cathodique du dispositif et se raréfient du côté anodique. On parle de polarisation de la concentration (**figure 3b**). Des facteurs d'enrichissement de 100 et de déplétion (raréfaction) de 500 ont été observés. Un modèle simple fondé sur des bilans de transfert de charge et des considérations sur une double couche électrique au sein du nanocanal a été proposé par les auteurs.

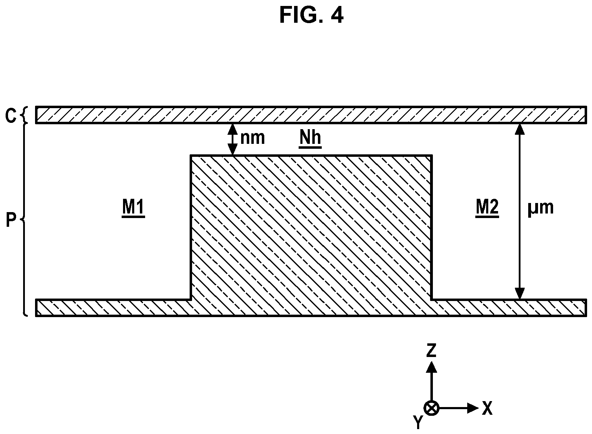

La compréhension de ce phénomène a cependant été nettement améliorée par le travail de Plecis et al. en 2008 [Plecis-2008]. Le dispositif étudié est cette fois constitué de deux microcanaux M1, M2 reliés par un nanocanal dit à fente horizontale Nh. On parle de dispositif Micro/Nano/Micro (MNM). Les canaux sont formés dans une plaque P s'étendant selon un plan XY et sont recouverts par une plaque de capot C, typiquement une lamelle. Les nanocanaux s'étendent selon l'axe X compris dans le plan XY. Un nanocanal à fente horizontale correspond à une portion de canal dont la profondeur, c'est à dire la dimension selon l'axe Z qui est orthogonal au plan XY, est inférieur à la profondeur des deux microcanaux de part et d'autre (voir **figure 4**).

Les solutions simulées sont constituées de fluorescéine diluée dans des solutions de KCl de forces ioniques variables.

Dans le cas particulier d'un dispositif en verre, chargé négativement en surface pour des pH supérieurs à 2, les anions présents en solution, du fait de la répulsion de parois, ne peuvent transiter avec le même débit que les cations à travers la nanofente. Il en résulte une répartition inégale de charges électriques de part et d'autre de la nanofente. Le champ électrique local varie donc, et avec lui le flux électrophorétique local. En certains points, il peut arriver que le flux électrophorétique compense le flux électro-osmotique. La vitesse des analytes chargés (ou des ions) y est nulle. Ces points d'annulation du flux (points focaux) peuvent être stables (les ions/analytes qui s'en éloignent y sont ramenés) ou instables (les ions/analytes qui s'en éloignent regagnent de la vitesse et sont emportés). Les ions/analytes s'accumuleront bien évidemment aux points focaux stables.

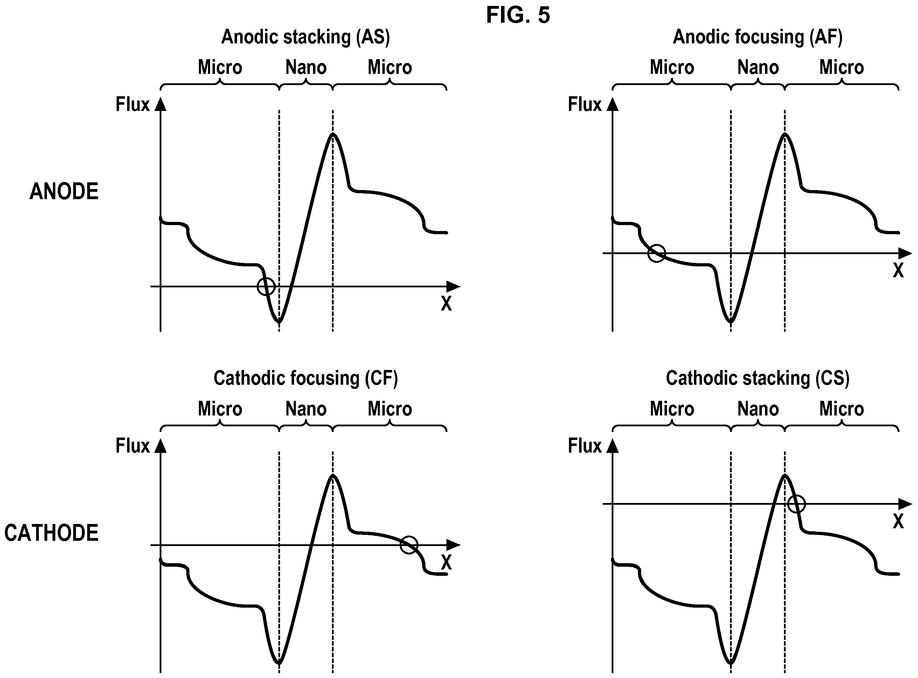

La **figure 5** illustre la forme du flux total (flux électro-osmotique + flux électrophorétique) de fluorescéine (Cᵢₙᵢₜᵢₐₗₑ=1nM) selon la position dans un dispositif Micro/Nano/Micro dans une solution de KCL (10µM). H_{micro}=2,5µm, Hₙₐₙₒ=50nm. Au point entouré sur la **figure 5****,** la vitesse des ions est nulle et ceux-ci s'accumulent.

On parlera de tâche de concentration ou point focal ou encore de spot pour désigner une accumulation d'analytes en une localisation particulière.

Selon la valeur du flux électro-osmotique, ce profil de flux se déplace sans déformation de haut en bas et ce point focal change de position. Si la tâche de concentration se trouve contre la nanofente on parlera de « *stacking* » ; si elle en est loin de « *focusing* ». Ce *stacking* et ce *focusing* peuvent être anodique ou cathodique.

Cela permet de définir quatre régimes de préconcentration : *Anodic Stacking* (AS), *Anodic Focusing* (AF), *Cathodic Focusing* (CF) et *Cathodic Stacking* (CS).

Ces résultats sont issus de [Plecis-2008]. Les termes anglais sont consacrés.

Un dispositif similaire de nanocanal à fente horizontal Nh a été utilisé par Louër et al. [Louer-2013]. Le dispositif intègre un nanocanal de 100µm de long et 150nm de profondeur dans un microcanal de 1µm de profondeur (cf. **figure 4**).

Le document WO2010034908 décrit lui aussi un nanocanal horizontal (voir figure 10b par exemple).

D'autres dispositifs existent pour générer la perturbation qui provoque l'électroconcentration.

Sung et al. [Sung-2012] ont développé un dispositif à base d'un canal situé entre deux réservoirs, obstrué par une membrane poreuse (membrane Nafion). Le canal a une largeur constante de 50µm. Une variante du dispositif consiste à utiliser différentes longueurs de réservoir pour constater des variations de la préconcentration.

Le document WO2010052387 présente un dispositif dont la perturbation est générée par un revêtement particulier polarisable.

En matière de séparation ou de concentration de protéines, les principales méthodes employées sont des techniques de chromatographie (exclusion, échange ion, affiné) ou bien d'électrophorèse sur gel (Western Blot) ou capillaire. L'analyse des protéines séparées se fait quant à elle avec différents moyens (dichroïsme, spectrophotométrie, RMN, spectrométrie de masse, rayon X). Des méthodes immunologiques peuvent aussi être utilisées (ELISA par exemple).

Néanmoins, ces solutions présentent plusieurs limitations.

Les spots générés de concentration ne sont pas toujours suffisamment précis, ce qui empêche de caractériser les molécules ou les particules.

L'architecture du dispositif n'est pas adaptée pour observer les spots, notamment dans une volonté d'analyse, de discrimination ou encore de tri de particules ou molécules.

Les nanofentes horizontales présentent une largeur qui ne favorise pas le développement de dispositif compact et performant.

Certains dispositifs nécessitent des tensions électriques importantes, ce qui les rend peu utilisables pour la fabrication de dispositifs efficaces et pratiques.

Les méthodes non liées à la préconcentration ne sont pas toujours rapides, efficaces et déplaçables. Les immunotests sur surface sont notamment sujets à des adsorptions non spécifiques qui faussent parfois les résultats.

Il existe donc un besoin de développer un dispositif performant pour discriminer des molécules ou des particules de façon efficace et rapide.

Il existe aussi un besoin concernant les moyens de perturbations pour générer l'électropréconcentration, qui soit efficaces, répétables, de faible dimension, etc.

### PRESENTATION DE L'INVENTION

L'invention propose un dispositif selon la revendication 1, un système selon la revendication 14 et un procédé selon la revendication 17 permettant de résoudre chacun au moins un des problèmes précités. Des modifications avantageuses sont définies par les revendications dépendantes.

### PRESENTATION DES FIGURES

D'autres caractéristiques, buts et avantages de l'invention ressortiront de la description qui suit, qui est purement illustrative et non limitative, et qui doit être lue en regard des dessins annexés, sur lesquels :
- la figure 1, déjà présenté, illustre le fonctionnement général de la compétition des flux,
- les figures 2, 3a, 3b, 4 déjà présentées, illustrent des dispositifs de l'art antérieur,
- la figure 5, déjà présentée, illustre les modes de préconcentration,
- les figures 6 et 7 illustrent un système dans lequel se positionne un dispositif conforme à plusieurs modes de réalisation de l'invention,
- la figure 8 illustre une vue du dessus d'un mode de réalisation d'un dispositif dit à fente verticale, conforme à l'invention,
- les figures 9a, 9b illustrent deux vues en coupe (longitudinales et orthogonales entre elles) d'un dispositif dit à fente verticale, conforme à l'invention,
- les figures 10a, 10b illustrent les deux mêmes vues en coupe d'un dispositif de l'art antérieur, dit à fente horizontale,
- la figure 11 illustre l'un mode de réalisation possible du dispositif avec deux canaux d'observation en série, conforme à l'invention,
- les figures 12, 13, 14a à 14c illustrent un dispositif conforme à un mode de réalisation dit à code-barres, conforme à l'invention,
- les figures 15a à 15c illustrent les résultats de préconcentration dans chacun des canaux d'un dispositif dit à code-barres, conforme à l'invention,
- les figures 16a à 16f représentent des photographies dont ont été issus les résultats des figures 15a à 15c,
- la figure 17 illustrent les étapes d'un procédé de détection/d'analyse/d'identification d'analyte conforme à l'invention,
- la figure 18 illustre la différence de positionnement des spots de concentration dans les différents canaux d'un dispositif dit à code-barres, conforme à l'invention,
- la figure 19 illustre le principe physique sous-jacent pour discriminer deux analytes à l'aide d'un dispositif à code-barres, conforme à l'invention,
- la figure 20, semblable à la figure 18, illustre la différence de positionnement des spots de concentration dans les différents canaux un dispositif dit à code-barres conforme à l'invention, pour deux analytes différents contenus dans un même électrolyte,
- la figure 21 illustre différentes couches pour fabriquer un dispositif conforme à l'invention,
- la figure 22 illustre les différentes étapes de fabrication d'un dispositif conforme à l'invention.

### DESCRIPTION DETAILLEE

Plusieurs aspects de l'invention vont être à présent décrits en détail.

Les dispositifs 10 concernés ici sont des dispositifs de détection/préconcentration sélectifs d'analytes chargés ou non contenus dans un électrolyte. Ces dispositifs permettent aussi de faire des réactions chimiques et biochimiques de manière très locale en mélangeant plusieurs groupes de molécules, généralement présentes en faible quantité.

Ces dispositifs sont de type Micro-Nano-Micro, ou MNM, c'est-à-dire, au sens le plus large, comprenant une succession d'au moins deux canaux dont les dimensions varient. Ces dispositifs 10 fonctionnent à l'aide de l'application d'une tension électrique à ses bornes, éventuellement couplée à une pression hydrodynamique.

Le dispositif 10 est configuré pour recevoir des électrolytes comprenant des analytes. On parlera de solution par la suite.

### Système général

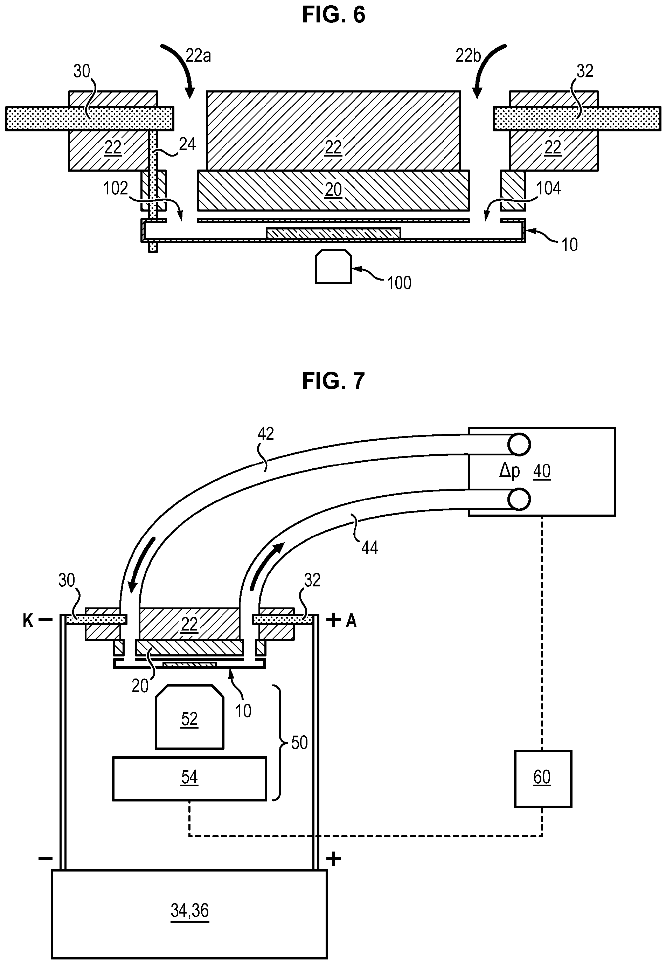

Les **figures 6 et 7** illustrent un système général dans lequel se positionne le dispositif.

Le dispositif 10 comprend un canal d'observation 100, de type MNM, au sein duquel se produit des phénomènes d'électropréconcentration. Le canal d'observation 100 s'étend selon une direction longitudinale X. Il peut se réaliser sous différents modes qui seront décrits par la suite.

En utilisation, le canal d'observation 100 est empli de solution. Le dispositif 10 comprend au moins deux ouvertures 102, 104, configuré pour permettre d'appliquer une différence de potentiel dans le canal d'observation 100 à l'aide d'électrodes 30, 32. Pour cela, chaque ouverture 102, 104 est en communication fluidique avec une des extrémités du canal d'observation 100.

Un mode de réalisation d'un système comprend le dispositif 10, un joint 20 typiquement en PDMS (polydiméthylsiloxane), un bloc 22 typiquement en PMMA (polyméthacrylate de méthyle), assemblés les uns sur les autres et maintenus en place par des moyens de serrage 24 tels que des vis. Le bloc en PMMA comprend deux ports 22a, 22b en regard des ouvertures 102, 104. Les ports 22a, 22b sont configurés pour être emplies de solution (par exemple à l'aide d'une seringue) et sont de taille suffisante pour accueillir chacune une électrode 30, 32 (par exemple une électrode en platine). Le joint 20, positionné entre le bloc 22 et le dispositif 10 a pour fonction d'étanchéifier la liaison, puisque le niveau de solution doit atteindre les électrodes qui ne sont pas dans le même plan que les ports 102, 104.

Plus que deux ports 22a, 22b et deux ouvertures 102, 104 peuvent être prévues, pour pouvoir multiplier le nombre d'électrodes formant anode et cathode. Cela permet un remplissage plus rapide et de simplifier le nettoyage.

Les électrodes 30, 32 sont reliées à un générateur de tension contrôlable 34 (**figure 7**) pouvant typiquement délivrer une différence de potentiel allant de quelques dizaines de volts à plusieurs centaines de volts. Les tensions qu'il doit pouvoir générer seront précisées par la suite en fonction des modes de réalisation du dispositif 10. Dans le cas des expériences menées pour la présente invention, le générateur couvrait une plage de -110V à +110V. Un certain nombre d'appareils électroniques 36 sont susceptibles d'être couplés au générateur afin de permettre un meilleur contrôle ou une meilleure mesure des tensions appliquées ou des caractéristiques électriques du système (impédance etc.).

Un des deux ports 22a, 22b correspond ainsi à l'anode A et l'autre à la cathode K.

Complémentairement, les ports 22a, 22b, ou au moins l'un d'entre eux, sont configurés pour recevoir un générateur de pression contrôlable 40. Ce générateur 40 permet d'appliquer une différence de pression, hydrodynamique, le long du canal d'observation 100. Par exemple, le contrôleur de pression 40 peut être relié à des tuyaux souples 42, 44 pouvant s'insérer dans ou encercler les ports 22a, 22b peuvent convenir.

Les valeurs des pressions sont déterminées conjointement aux valeurs de tensions électriques. Néanmoins, des pressions inférieures à 1 bar conviennent pour déplacer les spots et les rendre exploitables.

Un dispositif d'imagerie 50 est prévu dans le système, pour acquérir des vidéo/images du canal d'observation 100. Le dispositif 50 comprend généralement un microscope 52 couplé à une caméra 54. En fonction des longueurs d'onde, visibles ou non, que l'on souhaite observer, des filtres peuvent être utilisés et l'éclairage peut être adapté. Par exemple, une lampe à mercure avec des filtres peut être utilisée pour exciter et observer des analytes de type fluorescents (pour des longueurs d'onde dans le visible donc). Le dispositif d'imagerie 50 doit permettre d'acquérir des mesures d'intensité de fluorescence faible pour suivre la concentration en analytes. Le microscope 52 est généralement couplé à un obturateur (par exemple un *shutter* Uniblitz VCMD1) pour limiter le photo-blanchiment. Une méthode d'identification rapide consiste à lire optiquement la fluorescence.

Le pilotage du générateur de tension 34 et, le cas échéant, du générateur de pression 40 est effectué par une unité de calcul 60 qui peut aussi récupérer en direct les images acquises par le dispositif d'imagerie 50.

On définit au canal d'observation 100 une longueur Lo, selon l'axe longitudinal X et des sections orthogonales au dit axe X.

Le dispositif 10 comprend notamment trois parties : une partie réservoir où arrive la solution, une zone d'observation, reliée à la partie réservoir, où se produit l'électropréconcentration, et une zone de perturbation, reliée à deux zones d'observation de part et d'autre de la zone de perturbation. La zone d'observation et la zone de perturbation forment le canal d'observation 100. Dans le cas d'un dispositif MNM, la zone d'observation a généralement une section dont la dimension minimum est de l'ordre du micromètre, alors que la zone de perturbation a une section dont la dimension minimum est de l'ordre de la centaine de nanomètres. Néanmoins, cette appellation générique MNM ne saurait être limitative car elle peut englober des zones d'observation dont la dimension minimum est de l'ordre de la centaine de nanomètres.

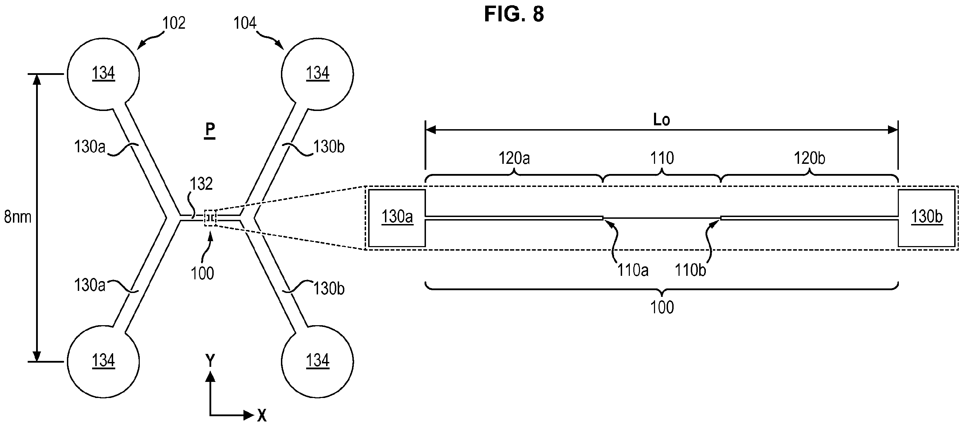

La **figure 8** représente un mode de réalisation général d'un dispositif 10.

Le dispositif comprend une plaque P définissant un plan XY. La direction longitudinale X est parallèle à ce plan XY. Un mode de réalisation de fabrication de la plaque sera détaillé par la suite. Il s'agit globalement de verre ou de silice, mais pas uniquement.

Le canal d'observation 100 est formé dans la plaque.

Le canal d'observation 100 comprend un nanocanal 110 comportant une première extrémité 110a et une deuxième extrémité 110b. Le nanocanal 110, comme indiqué précédement, forme la zone de perturbation.

Le canal d'observation 100 comprend un premier microcanal 120a relié à la première extrémité 110a du nanocanal 110 et un deuxième microcanal 120b relié à la deuxième extrémité 110b du nanocal 110. Les deux microcanaux 120a, 120b sont référencés 120 de façon générique.

Les trois canaux 110, 120a, 120bsont donc agencés en série et préférablement en disposition rectiligne. On peut alors définir une longueur totale Lo du canal d'observation 100.

Par microcanal, on entend un canal, c'est-à-dire une fente réalisée dans la plaque. Micro- et nano- se rapportent généralement et de façon non limitative à une dimension minimum de la section, qui est soit de l'ordre du micromètre, soit de l'ordre de la centaine de nanomètres. Plus précisément, nano- et micro- signifie qu'une dimension est de l'ordre de la centaine de nanomètres dans un canal et que cette même dimension est de l'ordre micrométrique dans un autre canal.

Un premier conduit d'amenée 130a, respectivement un deuxième 130b, est relié au premier canal d'observation 110a, respectivement le deuxième 110b. Les conduits d'amenée forment la partie réservoir précitée. Ils ont pour fonction de fournir un volume suffisant de solution pour permettre la bonne utilisation du dispositif 10.

En particulier, dans une géométrie particulière, les conduits d'amenée comprennent chacun deux réservoirs distincts, qui se rejoignent par le biais d'un canal intermédiaire 132 au niveau de l'extrémité du premier microcanal 120a, respectivement le deuxième microcanal 120b. Les dimensions des sections des conduits d'amenées sont généralement supérieures à 100µm pour fournir une quantité de solution suffisamment importante pour que la préconcentration se produise dans les microcanaux 120. Les canaux d'amenées 130a, 130b forment alors deux dièdres symétriques transversalement à l'axe longitudinal X, chaque dièdre étant symétrique par rapport à l'axe longitudinal X.

Les ouvertures 102, 104 sont formées en regard des conduits d'amenée 130a, 130b en général au niveau d'une zone 134 de dimension plus importante que la largeur des autres parties des conduits d'amenées 130a ,130b. En figure 8, on dénombre deux zones 134 (associées à deux ouvertures 102, 102 et 104, 104 et deux ports 22a, 22a, et 22b, 22b), symétriquement placées selon l'axe longitudinal X. A titre d'exemple, les deux zones 134 symétriques peuvent être situées entre 6 et 10mm l'une de l'autre. Dans le cas du dièdre précédent, les zones 134 sont aux extrémités desdits dièdres.

Lorsqu'empli de solution, il y a une continuité fluidique à travers des conduits d'amenée 130a, 130b (référencés plus simplement 130) et du canal d'observation 100.

Dans une section d'un canal, on définit une largeur I selon l'axe Y, c'est à dire la dimension du canal parallèlement au plan XY et orthogonal à l'axe longitudinal X, et on définit une profondeur P selon l'axe Z, qui est la dimension du canal orthogonalement à l'axe longitudinal X.

Les axes X et Y forment un angle direct dans le plan XY, et le trièdre XYZ est direct.

Les sections des canaux peuvent être rectangulaires mais cela implique des contraintes de nettoyage plus importantes (notamment à cause des zones stagnantes). Des sections avec des angles arrondis apportent plus de stabilité à la région de concentration et améliore la reproductibilité (car elle évite les effets de relargage issus des zones stagnantes). En particulier, ces sections dites arrondies forment des zones de transition entre les microcanaux 120 et le nanocanal 110.

Une plaque de capot C recouvre les canaux (canal d'observation 100 et d'amenée 130) pour isoler l'ensemble. Comme indiqué précédemment, des ouvertures 102, 104 sont prévus dans la plaque ou le capot pour permettre la communication fluidique.

Pour générer une perturbation, la section S110 du nanocanal 110 a une aire plus faible que celle de la section S120 du microcanal 120. Des grandeurs seront explicitées par la suite.

### Un dispositif 100 avec un canal d'observation à « fente verticale »

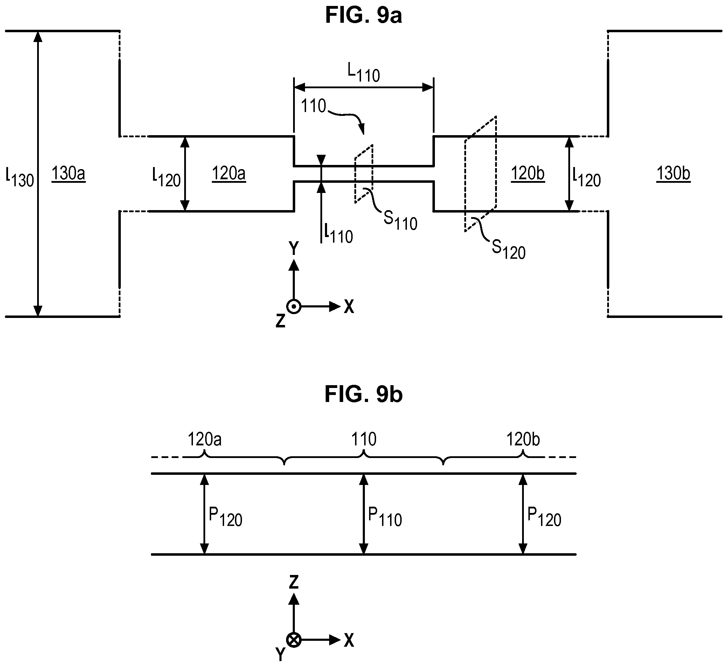

Les **figures 9a et 9b** représentent un mode de réalisation d'un dispositif 10 dit « à fente verticale ».

Le canal d'observation à fente verticale se définit par les dimensions des largeurs des microcanaux 120 et du nanocanal 110.

La largeur l120 des microcanaux 120 est comprise entre 1 et 20 µm, préférablement 1 et 10 µm. La largeur du nanocanal 110 est comprise entre 50 et 500 nm, préférablement entre 100 et 400 nm. On observe ainsi une restriction dans le plan XY, plus précisément selon l'axe Y, qui génère une perturbation. Pour des raisons de simplification de fabrication, la profondeur P selon l'axe Z est constante dans tout le canal d'observation 100, c'est-à-dire que la profondeur Pm des microcanaux 120 est la même que la profondeur P110 du nanocanal 110.

Une profondeur aux alentours de 1µm convient. Néanmoins une profondeur comprise entre 100 et 500nm peut aussi convenir, de sorte que les deux dimensions de la section du nanocanal soit de l'ordre de la centaine de nanomètre. Dans ce cas-là, le microcanal 120 a une dimension micrométrique et une dimension nanométrique et le nanocanal 110 a deux dimensions nanométriques.

Dans un mode de réalisation, la largeur l120 et la longueur du premier microcanal 120a sont égales à respectivement à la largeur et la longueur du deuxième microcanal 120b. Cette caractéristique permet d'avoir un dispositif symétrique, ce qui est important si l'on souhaite s'affranchir des caractéristiques d'architectures du dispositif 10 pour discriminer deux analytes qui se concentrent chacun dans un des microcanaux.

Dans un autre mode de réalisation, il peut être prévu expressément d'avoir des largeurs différentes de microcanaux l120. Cela permet d'avoir un facteur supplémentaire de discrimination, notamment pour des électrolytes complexes, c'est-à-dire des électrolytes comprenant une pluralité d'analytes différents avec des tailles et des groupements chimiques différents.

Complémentairement ou alternativement, la longueur du premier microcanal 120a peut être différente de la longueur du deuxième microcanal 120b.

Le dispositif en fente verticale permet de concentrer d'un facteur 1000 des analytes.

### Un dispositif 100 avec un canal d'observation à « fente horizontale »

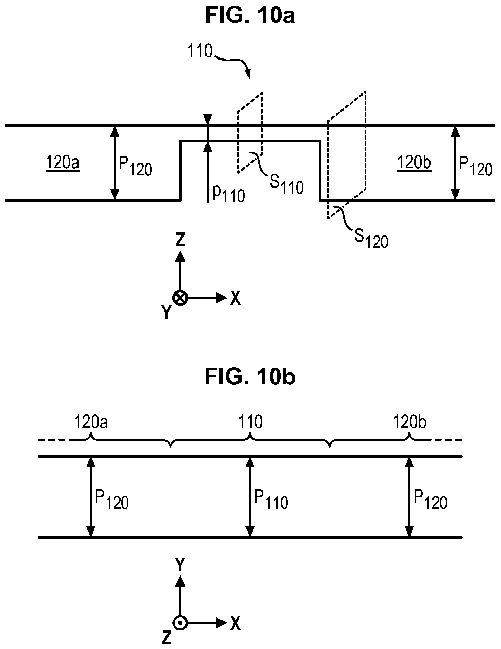

Les **figures 10a et 10b** représentent un mode de réalisation d'un dispositif 100 dit « à fente horizontale ». Ces dispositifs ont été présentés en introduction et sont déjà connus.

A l'inverse du canal d'observation 100 à fente verticale, la largeur du microcanal l120 est égale à la largeur selon l'axe Y du nanocanal l110. En revanche, la profondeur selon l'axe Z du nanocanal P110 est inférieure à la profondeur du microcanal P120. Par exemple, la profondeur du nanocanal P110 est comprise entre 75 et 350 nm alors que la profondeur du microcanal P120 est comprise entre 2 et 3µm, par exemple aux environ de 2,5µm.

### Comparaison entre les « fentes verticales » et les « fentes horizontales ».

Grâce à la « fente verticale », le phénomène de pré-concentration est visible selon une direction orthogonale à la dimension nanométrique, c'est-à-dire la largeur du nanocanal 110, facilement accessible pour le dispositif d'imagerie 50. En outre, la surface totale du dispositif peut être réduite (voir notamment le dispositif à code-barres ci-dessous), ce qui permet d'obtenir plus de résultats à taille de dispositif équivalente. Cela permet d'obtenir plus de résultat pour une seule et même expérience, ce qui évite les contingences liées à la répétabilité et permet un gain de temps et de matériel : les paramètres idéaux peuvent être trouvés en une même manipulation.

### Complément sur les microcanaux

Dans un mode de réalisation particulier, qui s'applique à tout canal d'observation 100 à fente verticale ou horizontale, seul un microcanal 120a ou 120b est prévu. Par conséquent, le nanocanal 110 est relié de l'autre côté directement avec le canal d'amenée 130. En série, on a ainsi l'agencement suivant : canal d'amenée 130a, microcanal 120a, nanocanal 110, canal d'amenée 130b.

Ce mode de réalisation, quoique non symétrique, peut être avantageusement utilisable avec l'application d'une pression hydrostatique qui permet de faire déplacer la tâche de concentration.

La présente description a été faite pour deux microcanaux 120a, 120b mais s'applique au dispositif avec un seul microcanal.

### Un dispositif 100 avec deux canaux d'observation en série.

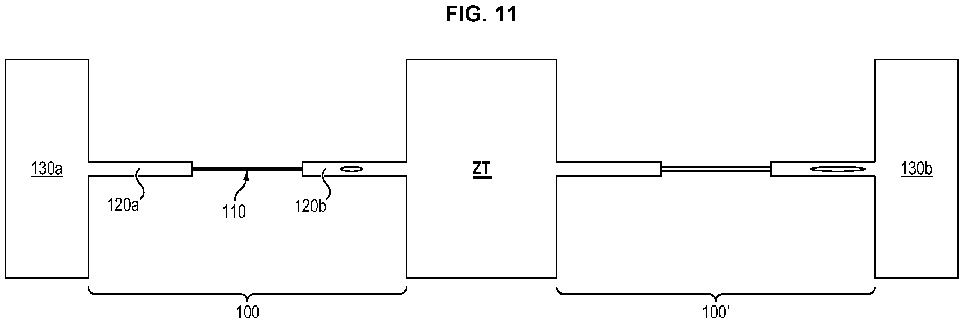

Dans un mode de réalisation illustré en **figure 11****,** le canal d'observation 100 est dit primaire et le dispositif comprend au moins un autre canal d'observation dit secondaire, situé entre le canal d'observation primaire 100 et un des canaux d'amenée 120a, 120b, de sorte que les canaux primaires 100 et secondaires 100' sont agencés en série. Une zone tampon ZT est prévue entre les deux canaux d'observation 100, 100'.

Plus de deux canaux d'observation 100, 100' peuvent ainsi être prévus en série.

Les canaux d'observation primaire 100 et secondaires 100' peuvent être identiques en matière de dimensions, pour des raisons de symétrie. Alternativement, avoir des canaux d'observation primaire 100 et secondaire 100' différents permet d'observer davantage de comportements différents pour les analytes présents dans l'électrolyte.

La pression hydrodynamique peut en outre être appliquée au niveau de la zone ZT, en remplacement ou en complément des autres endroits d'application décrits précédemment. Le dispositif doit alors être adapté, notamment en matière d'ouverture, de ports, et d'étanchéité.

Plusieurs arrangement de canaux primaires et secondaires 100, 100' en série peuvent être prévues en parallèle, en partageant un unique canal d'amenée 130a, un unique canal d'amenée 130b et, accessoirement, une unique zone tampon. Cette configuration avec plusieurs canaux d'observation en parallèle est appelée « code-barres ».

### Un dispositif 10 à « code-barres »

Le canal d'observation à « fente verticale » est particulièrement adapté pour un dispositif 10 dit « à code-barres ». En effet, ce dernier est commode à fabriquer et génère des spots localisés Sp.

Néanmoins, un dispositif à fente horizontale peut aussi être utilisé.

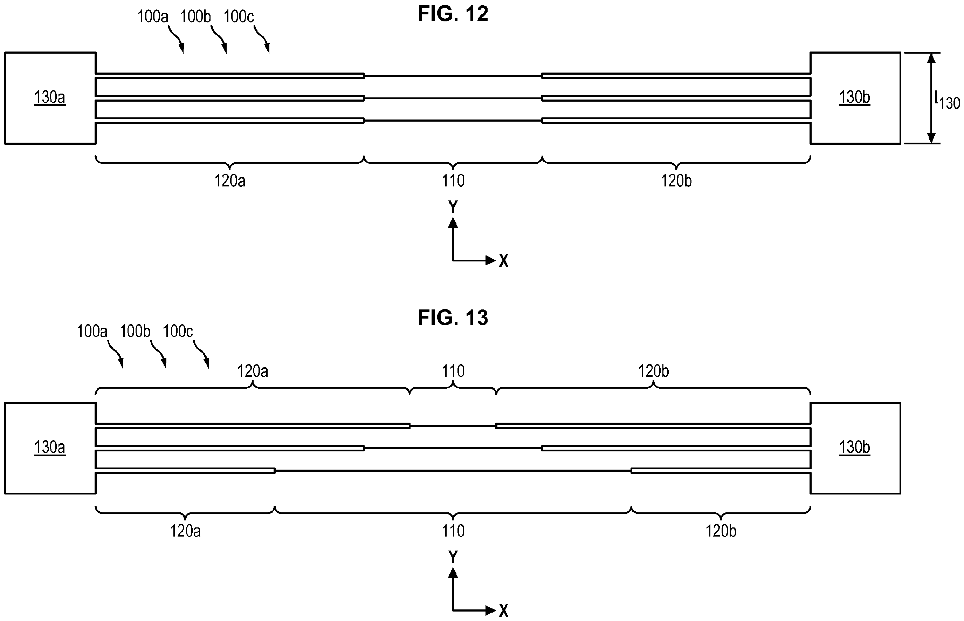

En référence aux **figures 12 à 13** notamment, le dispositif 10 à code-barres comprend une pluralité de canaux d'observation 100a, 100b, ... s'étendant chacun selon une direction longitudinale X dans un plan XY. Préférablement, pour simplifier les mesures, pour simplifier l'architecture et pour simplifier la fabrication, les canaux d'observation 100a, 100b sont parallèles entre eux.

Dans ce dispositif 10 à code-barres, pour au moins deux nanocaux, une dimension parmi leur longueur respective ou leur section S110 respective est différente. En d'autres termes, tous les nanocanaux 110 n'ont pas la même longueur ou la même section S110.

Préférablement, pour obtenir un maximum de mesures possibles en une seule expérience, il existe un seul nanocanal 110 vérifiant un couple longueur/section donné, ou alors le nombre de redondances, c'est-à-dire de canaux d'observation qui ont les mêmes caractéristiques, est limité.

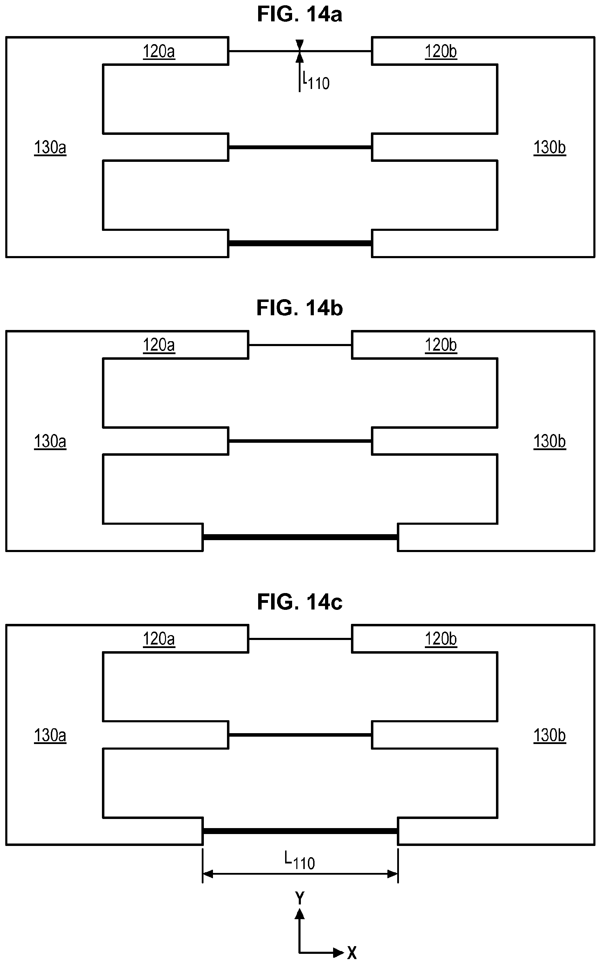

Par exemple, si le dispositif 100 à code barre comprend deux canaux d'observations 100a, 100b à fente verticale on pourra avoir les configurations suivantes :
- L(100a) = L(100b), I(100a) ≠ I(100b) (**figure 14a**), ou
- L(100a) ≠ L(100b), I(100a) = I(100b) (**figure 14b**), ou
- L(100a) ≠ L(100b), I(100a) ≠ I(100b) (**figure 14c**).

Dans chacun des canaux d'observation 100, et plus particulièrement, chaque microcanal 120a, 120b de chacun des canaux d'observation 100, la préconcentration est susceptible d'être observée.

Préférablement, la longueur Lo est identique pour tous les canaux d'observation 100 afin de simplifier la fabrication de dispositif 100. Néanmoins, si besoin, une condition peut être l'égalité de longueur des microcanaux 120 (notamment lorsque les nanocanaux 110 ont des longueurs différentes).

Les canaux d'observation sont espacés deux à deux d'une distance comprise entre 15 et 100µm, préférablement entre 30 et 50µm. Plus cette distance est faible, et plus le dispositif peut être compact ou intégré un nombre important de canaux d'observation 100.

Les canaux d'amenées 130a, 130b jouent ici un rôle important de tampon et de réservoir. En effet, il est important que chaque canal d'observation 100 puisse être considéré comme indépendant et qu'il n'y ait aucun mélange entre les microcanaux 120a ,120b. En d'autres termes, cela signifie que le volume de solution présent dans les canaux d'amenée est considéré comme infini d'un point du vue théorique. Une valeur de 100 µm de largeur l130 convient. Une profondeur égale à la profondeur des microcanaux 120 convient.

Le phénomène de préconcentration dépend de plusieurs facteurs, dont notamment la perturbation générée dans le canal. Il s'agit ici du nanocanal. En modifiant les largeurs (et donc la section) et/ou la longueur, les spots de préconcentration Sp s'en trouvent modifiés. Comme la position, le mouvement, la largeur, l'intensité de chaque spot de préconcentration est une information susceptible de renseigner sur l'analyte observé, la pluralité de canaux d'observation 100a, 100b, 100c permet d'acquérir une multiplicité d'informations en une seule expérience.

En particulier, la préconcentration, le tri et la séparation de différentes molécules mélangées sont facilités par ce dispositif à code-barres qui permet d'explorer simultanément les effets de nombreux jeux de paramètres.

Cela permet en outre de trouver rapidement les paramètres convenables pour effectuer une expérience dont les résultats sont exploitables.

Comme indiqué précédemment, l'analyse des comportements des spots Sp sur les différents canaux d'observation 100 permet de dresser une identification de l'analyte.

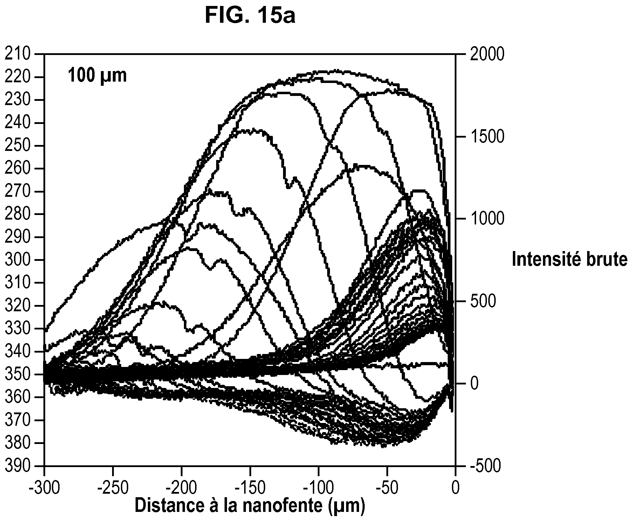

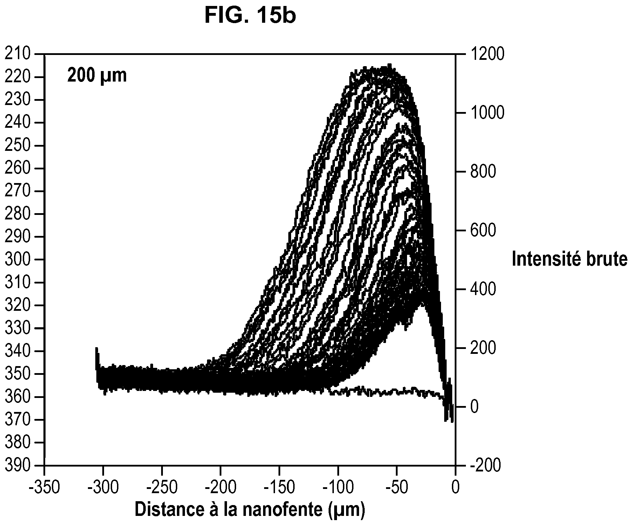

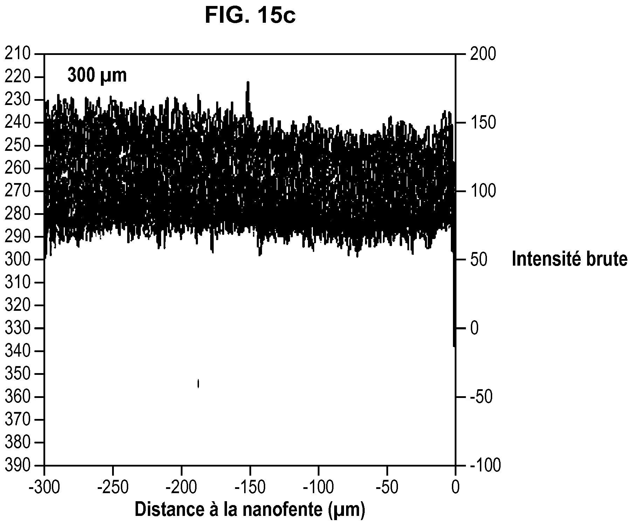

Les **figures 15a****,** **15b****,** **15c** illustrent le comportement d'un analyte dans les différents canaux d'observation en fonction du temps, pour une variante conforme à la **figure 14a****.** On remarque :
- pour I110=100nm, une préconcentration instable, avec un spot qui se déplace dans le microcanal 120 (**figure 15a**),
- pour I110=200nm, une préconcentration sable, avec un spot fixe (**figure 15b**),
- pour 1110=300 nm, une absence de préconcentration (**figure 15c**).

### Réalisation d'une expérience

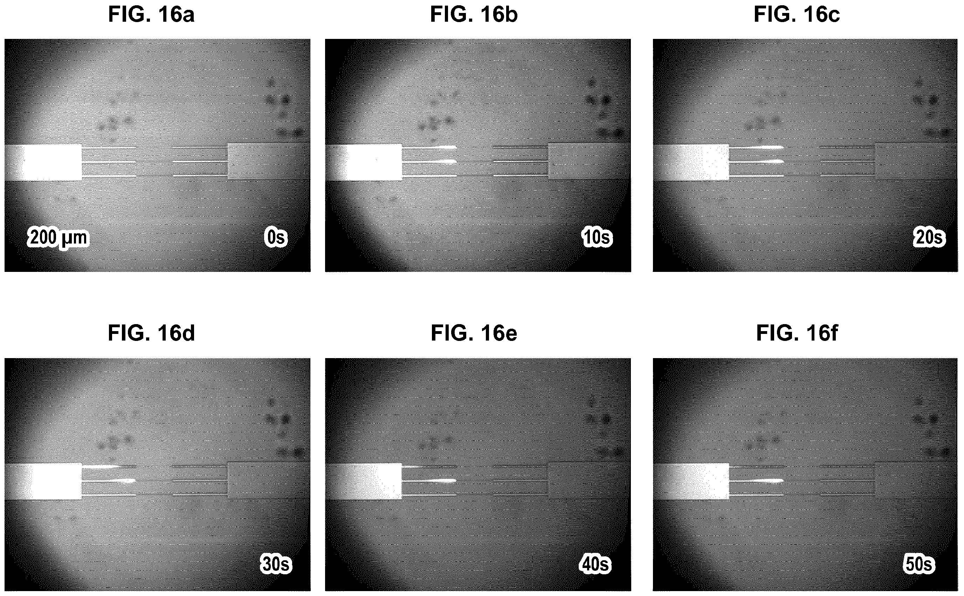

Le dispositif 10 est mis en place au sein d'un système tel que décrit précédemment. Le dispositif est rempli par ses ouvertures 102, 104, via les ports 22a, 22b, avec une solution ionique dont la composition ou la force ionique peuvent être adaptées selon les cas. Une fois le remplissage effectué, on applique des tensions électriques et éventuellement des pressions hydrodynamiques au sein du dispositif. Les **figures 16a à 16f** montrent une préconcentration réalisée avec une solution de fluorescéine de sodium à 10µM dans une solution de KCl à 10µM. Ce dispositif possède trois nanofentes de largeur différente de 100, 200 et 300 nm (de haut en bas sur les photos). Une tension de 20 V a été appliquée ainsi qu'une pression de 0,2 bar dans le sens opposé au flux électro-osmotique. Le temps d'observation est de 50s. La préconcentration pour ces paramètres s'effectue bien au sein de la zone intermédiaire, c'est-à-dire dans les microcanaux intermédiaires 120 a et 120b, prévue pour cela.

Cette expérience met en évidence l'intérêt de la pluralité de fentes à section différente, en l'espèce de largeur différente. En une seule mise en œuvre du protocole, trois préconcentrations sont réalisées, ce qui permet de déduire quelle largeur, compte tenu des autres paramètres, conduit à une préconcentration nulle ou instable, ou à l'inverse quelle largeur conduit à préconcentration stable, qui est généralement la donnée la plus facilement exploitable.

En relevant la stabilité et/ou les durées associées et/ou la position et/ou les vitesses de migration et/ou l'intensité du spot et/ou sa largeur et/ou sa forme, la matrice de paramètres de l'analyte peut être constituée.

### Rôle de la pression

L'application d'un gradient de pression à l'aide du générateur de pression contrôlable 40 permet de modifier les conditions de préconcentration.

En effet, l'ajout d'un flux de pression en sus des flux electro-osmotique et électrophorétique modifie le profil du flux total d'ions et le spot se déplace sous son influence. Louër et al. [Louer-2013] ont montré que l'ajout d'un flux de pression peut stabiliser un comportement instable (dont l'intérêt pour caractériser un analyte est moindre) ou changer le comportement global de l'ion observé.

Il est ainsi possible de préconcentrer un analyte (BSA dans la publication) dans une solution (tampon borate) tout aussi bien du côté anodique que du côté cathodique selon la pression appliquée.

Pour identifier les analytes par l'observation de codes-barres constitués des tâches (ou spots) de préconcentration et de leur comportement, les possibilités offertes par l'ajout d'une pression hydrodynamique sont intéressantes puisque différents analytes/ions ne modifieront nécessairement pas leur comportement de la même manière selon la pression appliquée. Un code-barres pour chaque pression peut-être observé. Par ailleurs une préconcentration stable est toujours plus aisée à observer, et comme nous l'avons vu la pression est susceptible de stabiliser des comportements instables.

### Procédé de détection/préconcentration sélective

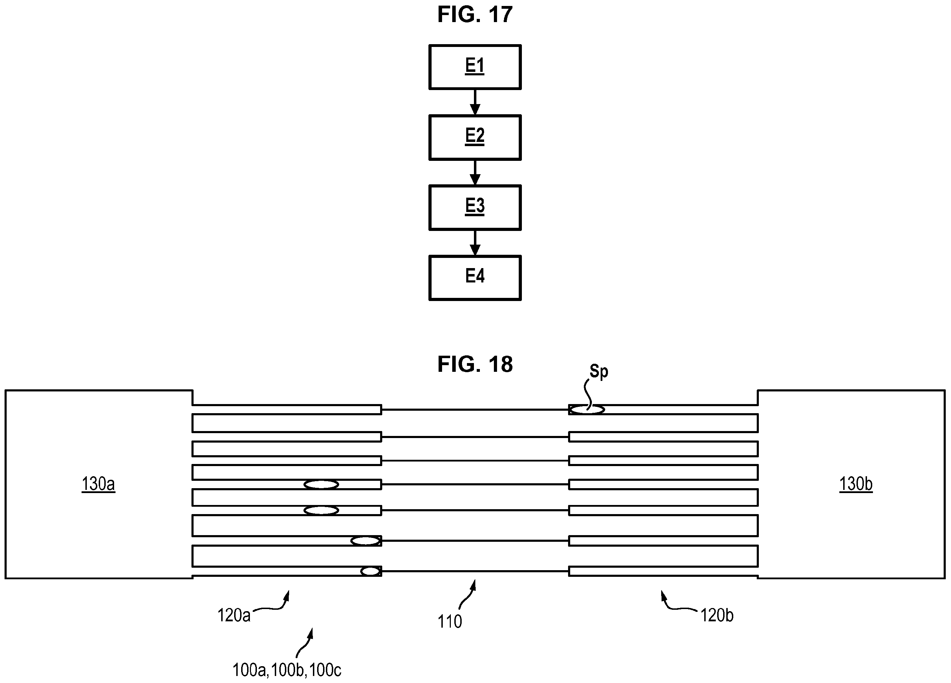

A l'aide du dispositif 100 à code-barres, plus précisément à l'aide d'un système comprenant un tel dispositif, il est possible de mettre en œuvre en procédé de détection ou d'identification, ou bien encore un procédé de préconcentration sélective, d'analytes chargés contenus dans un électrolyte (**figure 17**).

Dans une première étape E1, on emplit les canaux d'amenée 130, les micronaux 120 et les nanocanaux 110 avec de la solution. Ce remplissage peut se faire par injection via une seringue.

Dans une deuxième étape E2, des électrodes 30, 32 sont positionnées dans les ouvertures 22a, 22b du système et une tension est appliquée à l'aide de la source de tension contrôlable 34. La tension est appliquée durant une durée déterminée dans le protocole. Sous l'effet de la tension, des phénomènes de préconcentration peuvent se produire, ce qui génère l'apparition de sports de concentration d'analytes dans les microcanaux 120.

Complémentairement, pour les raisons explicitées précédemment, une pression hydrodynamique peut être appliquée sur un des ports 22a, 22b à l'aide du générateur de pression contrôlable 40.

Dans une étape E3 de mesure et d'analyse, on acquiert au moins une donnée parmi les données suivantes, pour au moins deux canaux d'observation 100a, 100b (voir **figure 18**) :
- position longitudinale dans le microcanal du spot de concentration Sp (ou son centre, ou une de ses extrémités, ou les deux),
- taille du spot Sp,
- intensité du spot (en fluorescence par exemple),
- vitesse de migration du spot,
- largeur du spot.

Cette liste n'est pas exhaustive. En particulier, on cherche à acquérir le plus de mesures possibles. Aussi est-il préférable d'acquérir le plus de données différentes possibles parmi les données précitées.

Chaque paramètre supplémentaire permet d'affiner la matrice et est donc susceptible de réduire l'ensemble des molécules pouvant lui correspondre.

Préférablement, les mesures sont acquises pour chaque canal d'observation, dont les caractéristiques physiques (longueur, largeur, profondeur) sont connues.

Dans une étape E4, les données obtenues et/ou traitées sont comparées avec une base de données. Pour cela, les données peuvent être agencées en matrice par exemple.

En comparant avec une base de données, par exemple les écarts entre les données, on peut caractériser ou identifier l'analyte, de sorte qu'au moins une information relative à l'analyte peut être obtenue. En outre, en fonction de l'application ou non d'une pression hydrodynamique, la cartographie peut changer. On peut ainsi obtenir une cartographie sans pression et une cartographie avec différentes pressions.

La génération de la base de données se fait par étalonnage sur plusieurs solutions modèles contenant des analytes connus.

Grâce à ce dispositif, on obtient une cartographie unique de l'analyte.

### Identification avec plus d'un types d'analyte

Dans certains cas, la solution ne contient pas un seul type d'analyte mais plusieurs. Le dispositif 10 à code-barres permet de sélectionner et de discriminer ces analytes.

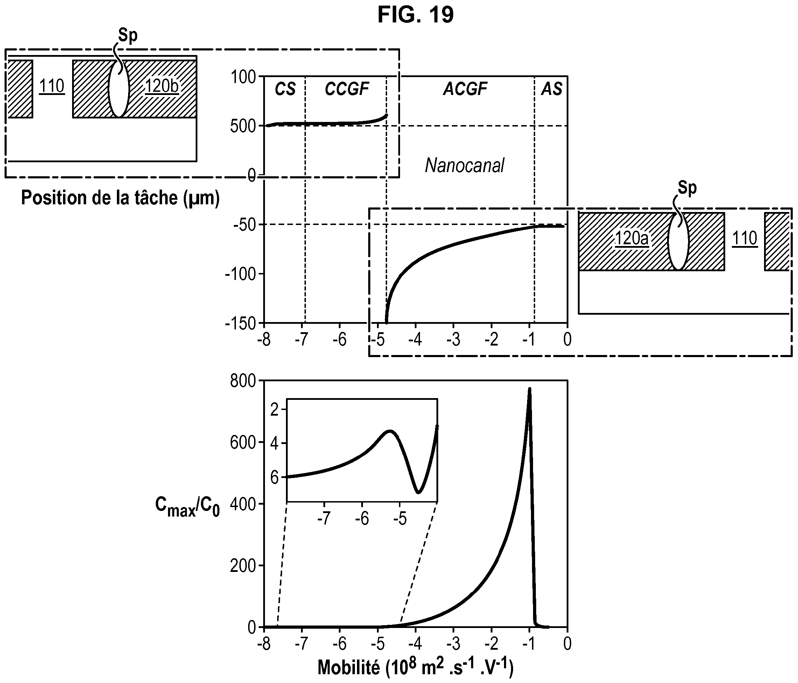

La **figure 19** illustre le fondement physique. En effet, une molécule ou une protéine ayant une grande mobilité sous champ électrique a tendance à se concentrer du côté cathodique K, alors qu'une molécule ou une protéine de faible mobilité sous champ a tendance à se préconcentrer du côté anodique A. On peut citer respectivement la fluorescéine et le sérum bovin d'albumine.

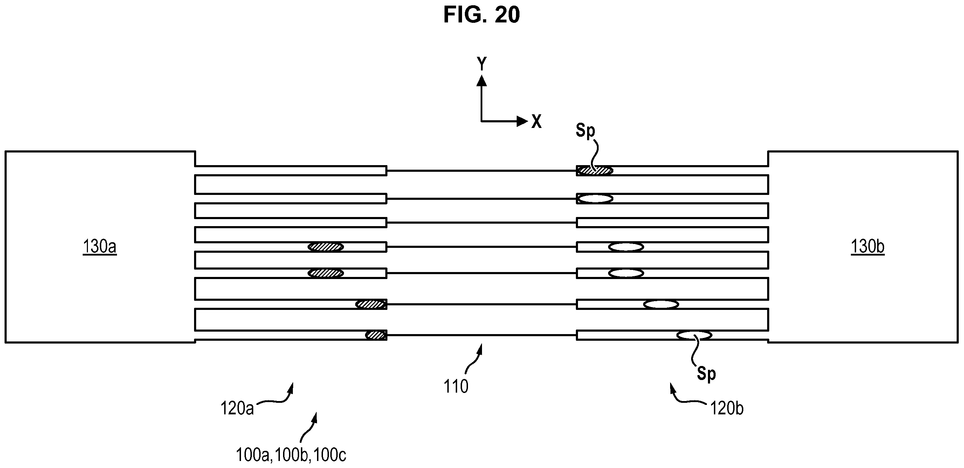

Si les mobilités sont suffisamment distinctes et que les spots relatifs à chaque type d'analyte sont bien distincts, comme illustré en **figure 20** (les spots de concentration sont illustrés par des ronds blancs et des ronds noirs, correspondant aux deux types d'analyte), il est même possible d'obtenir la matrice de chaque analyte en une seule manipulation. En fonction des analytes concernés, le générateur de pression contrôlable 40 peut alors jouer un rôle important pour que le dispositif 100 soit efficace sur les analytes.

Grâce à ce dispositif, on peut identifier chaque analyte existant au sein d'une solution contenant un mélange complexe.

### Détails sur la plaque du dispositif

La plaque P du dispositif (à fente verticale et/ou code-barre) comprend avantageusement une couche de silicium ou de verre et une couche de dioxyde de silicium SiO₂, dans laquelle est gravé le canal d'observation. La couche de SiO₂ est plus pure que le silicium ou le verre et sa gravure plus aisée à maitriser que celle du verre.

La plaque de capot C, généralement sous la forme d'une lamelle, qui recouvre les canaux peut être en verre.

### Application et domaines

Les applications sont variées et ont été décrites précédemment. Grâce à sa capacité de concentration, le dispositif à code-barres, en particulier celui à fente verticale, permet ainsi d'analyser des solutions contenant des biomolécules à l'état de trace ou d'ultra-trace, comme des agents pathogènes de la menace et du biorisque.

Les analytes peuvent être : des particules, des nanoparticules, des virus, des protéines, etc.

Les domaines concernés sont : la recherche scientifique, la gestion des risques chimiques et bactériologiques, la surveillance des polluants, le contrôle qualité des médicaments, la détection de marqueurs de maladies dans des liquides biologiques.

### Procédé de fabrication d'un dispositif à fente verticale

La fabrication d'un dispositif 100 à fente verticale nécessite de graver des tranchées de largeurs variables dans un matériau. Cette tranchée doit avoir une largeur contrôlée et une profondeur relativement grande afin d'autoriser un débit fluidique raisonnable. Une profondeur de 1µm convient, mais n'est pas limitative. En particulier, toute profondeur comprise entre 0,8µm et 5µm convient, et préférablement entre 1,8µm et 1,2µm.

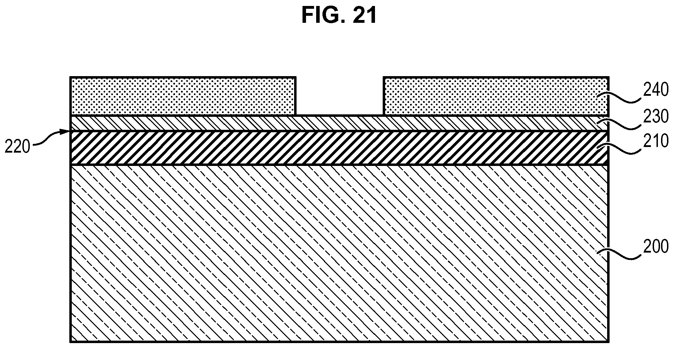

On désigne par substrat l'assemblage des différentes couches qui sont traitées pour fabriquer le dispositif 100 (voir **figure 21**). Ce terme est générique et s'applique aux assemblages à toute étape.

Le procédé décrit s'applique donc pour un dispositif comprenant une seule fente verticale ou une pluralité de fentes verticales en série, ou bien encore pour un dispositif « code-barres » comprenant une pluralité de fentes verticales en parallèle et éventuellement en série.

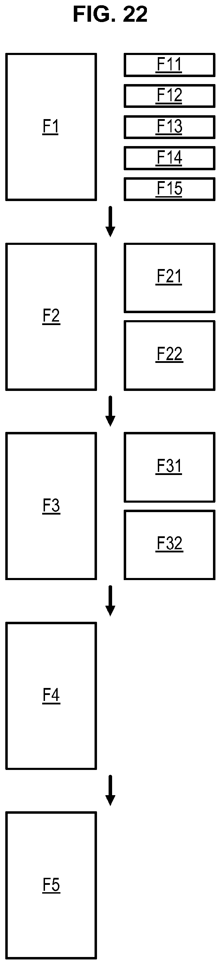

Le procédé de fabrication distingue plusieurs grandes étapes **(****figure 22****)** :
- une étape F1 de préparation du substrat,
- une étape F2 de gravure,
- une étape F3 de nettoyage.

Diverses étapes, groupées sous le terme générique d'étapes supplémentaire F4, vont aussi être décrites. Enfin, une étape d'assemblage F5 sera décrite.

Dans un premier temps F11, sur une plaque de verre ou de silicium 200, une couche de dioxyde de silicium SiO₂, référencée 210, est déposée. L'épaisseur de cette couche 210 dépend notamment de la profondeur souhaitée pour le canal d'observation 100. En l'espèce, une épaisseur de 1µm convient. La couche est déposée par dépôt chimique en phase vapeur assisté par plasma (PECVD). L'épaisseur de la plaque de verre ou de silicium n'a pas d'impact direct sur la préconcentration. En revanche, l'épaisseur doit être choisie pour permettre l'observation des tâches de préconcentration (distance focale de l'objectif, absorption du verre). Cette plaque est aussi thermalisée pendant la gravure subséquente, ce qui peut modifier les paramètres de gravure.

Le dioxyde de silicium est plus tendre et plus pur que le silicium et le verre. Par conséquent, il s'agit de cette couche 210 qui sera gravée.

Dans un second temps F12, une fine couche de germanium 220, de l'ordre du nanomètre, est déposée sur le SiO₂.

Dans un troisième temps F13, une couche épaisse d'aluminium 230 est déposée. Son épaisseur est comprise entre 150 et 250 nm, et est préférablement de 200 nm. La couche intermédiaire de germanium a uniquement pour effet de réduire la rugosité de la couche d'aluminium.

Dans un quatrième temps F14, l'aluminium est recouvert d'une couche de résine électrosensible positive 240, de type « ZEP520A ». Cette couche 240 a une épaisseur comprise entre 400 et 600 nm, et est préférablement de 500 nm. Cette résine est par exemple déposée par enduction centrifuge (*spin-coating*) sur la surface à 2000 tr/min pendant 30s (500 tr/min/s d'accélération), puis recuite 30 min à 160°C.

Dans un cinquième temps F15, la résine électrosensible est insolée en lithographie électronique, c'est-à-dire à faisceau d'électrons, afin de dessiner les motifs qui seront gravés. La résine est développée pendant 1min15s dans un développeur (par exemple le ZED-N50). L'ensemble peut être rincé à l'isopropanol.

Les motifs ouverts dans la résine exposent l'aluminium. Le substrat est alors prêt pour l'étape de gravure F2.

Dans un premier temps F21, l'aluminium est gravé avec un plasma chlore par ICP-RIE (torche à plasma et gravure ionique réactive) pour faire apparaitre le motif sur le SiO₂. Les caractéristiques employées ont été les suivantes : ∼35s, 5mTorr, Cl2 (20 sccm), bias:-100V, ICP: 500W. Une fois l'aluminium gravé par ce moyen, le motif apparait en SiO2.

Dans un deuxième temps F22, le SiO₂ est alors gravé avec un plasma à base de fluor (SF6), d'oxygène et d'argon. Plusieurs paramètres sont réglables dans un plasma ICP-RIE : l'énergie injectée dans le plasma (ICP) et celle transmise aux ions pour qu'ils bombardent la surface (le « *bias* »), ainsi que les proportions des gaz. En l'espèce, dans un mode de réalisation on n'active le bombardement ionique qu'une partie du temps (30-50% sur 500ms), afin que le plasma sans bombardement, plus isotrope, vienne lisser les parois des motifs gravés, et que les charges déposées en surface par le bombardement soient évacuées. Ces dernières en effet conduisaient à une gravure plus prononcée dans le voisinage des parois. Les caractéristiques employées ont été les suivantes : ∼13min, 5mTorr, SF6 (60 sccm), O2 (3 sccm), Ar (7 sccm), ICP: 300W, bias: 30W.

En particulier, pour le dispositif à fente verticale, l'étape de gravure consiste à graver l'ensemble des canaux en une seule étape. Les dimensions des micro- et nanocaux ont été présentées précédemment. En l'espèce, la profondeur de gravure est idéalement la même dans le micro- et le nano-canal.

La gravure est arrêtée avant d'arriver à la fin de la couche de dioxyde de silicium. Idéalement, la gravure est configurée pour s'arrêter au niveau de la fin de la couche. Le motif est alors terminé. L'ensemble est alors prêt pour l'étape de nettoyage F3.

Dans un premier temps F31, le restant de résine est retiré par un lavage, typiquement à l'acétone et au trichloréthylène.

Dans un second temps F32, le métal non gravé, car protégé par la résine, est retiré, typiquement par dissolution à la soude ou au mélange piranha (H₂SO₄(50%)/H₂O₂(50%)).

A l'issue de ces étapes F1, F2, F3, on obtient une plaque creusée par des tranchées qui forment les canaux.

Les étapes supplémentaires F4 sont alors prévues.

Dans un premier temps, le côté de la plaque de silicium ou de verre opposé à la couche de SiO₂, est couvert d'une résine, de type AZ5214. Cette couche a pour objectif d'éviter que des débris ne restent piégés dans la nanofente.

Dans un second temps, on perce les couches du substrat pour générer les ouvertures 102, 104 qui permettent l'injection de solution dans les canaux. Le perçage se fait de façon traversante. Les ouvertures 102, 104 doivent donc déboucher en regard des canaux d'amenée. Les ports sont ouverts dans le substrat à la micro-bilieuse ou à la perceuse. Dans le cas d'un perçage à la micro-bilieuse, on utilise un guide en métal, c'est-à-dire un bloc d'acier percé de trous. Ces trous sont disposés de façon à pouvoir être alignés avec tous les ports du dispositif. Le dispositif est collé à ce bloc, les trous en face des parties du motif que l'on souhaite percer. Un jet de microbille est projeté de l'autre côté du guide et vient éroder le verre ou le silicum au niveau des orifices du métal jusqu'à son perçage complet.

Dans un troisième temps, une fois les ports percés, le substrat est nettoyé au trichloéthylène, à l'acétone, à l'eau, à l'isopropanol et au au mélange piranha.

Le substrat est alors prêt pour être recouvert.

Dans une cinquième étape F5, le substrat, du côté des canaux gravés, est refermé à l'aide d'un capot C constitué typiquement d'une lamelle de verre. Le collage de cette lamelle peut être effectué de diverses manières : un collage thermique (mise en contact du substrat et du capot, sous vide, sous forte pression et température). Un mode de réalisation préférentiel utilise une résine comme couche intermédiaire de collage, le silsesquioxane d'hydrogène (HSQ), en employant alors le procédé décrit dans le document FR 1054183.

Le dispositif 10 est alors prêt à être monté dans le système présenté précédemment, permettant l'injection de liquide et l'application d'une tension électrique.

Afin d'éviter l'élargissement des fentes au cours de la gravure, une gravure sèche au plasma est préférée à une gravure humide. Les ions constituant le plasma sont accélérés à l'aide d'un champ électrique et viennent bombarder la surface à graver perpendiculairement à celle-ci. La gravure s'effectue donc préférentiellement selon cette direction. Une gravure sèche est donc susceptible de présenter une certaine anisotropie. Au cours d'une gravure humide par contre les réactions chimiques s'effectuent indépendamment de la direction et le substrat est gravé de façon isotrope.

Du fait de la conception des canaux en fente verticale, la gravure se fait seulement en deux étapes (gravure du métal et gravure du dioxyde de silicium) réalisables successivement sur une même machine.

Inversement, un dispositif qui contiendrait plusieurs canaux à fentes horizontales de profondeur différentes nécessiterait un masque pour chaque profondeur de canal et donc d'effectuer une pluralité de lithographie. En outre, l'encombrement est plus important puisque les nanocanaux à fentes horizontales ont une dimension micrométrique dans le plan XY. Or, un champ trop large, par exemple dans le cas de l'acquisition par le dispositif d'imagerie 50 avec photodiode, peut générer des complications.

Le procédé de fabrication relève de la nanolithographie électronique et de la gravure.

### REFERENCES

[Pu-2004] : Pu et al. Nano Letters, 2004, DOI : 10.1021/nl0494811
[Plecis-2008] : Plecis et al. Anal. Chem. 2008, DOI : 10.1021/ac8017907
[Louer-2013] : Louër et al. Anal. Chem. 2013, DOI: 10.1021/ac4016159
[Sung-2012] : Ko et al. Lab on a Chip 2012, DOI : 10.1039/c2lc21238b

## Revendications

1. Dispositif (100) de détection/préconcentration sélective d'analytes chargés contenues dans un électrolyte, comprenant :
une pluralité de canaux d'observation (100a, 100b, ...) s'étendant chacun selon une direction longitudinale (X) dans un plan (XY),
deux canaux d'amenée (130a, 130b) reliées entre eux par la pluralité de canaux d'observation (100) et configurés pour amener l'électrolyte dans ladite pluralité de canaux d'observation (100), chaque canal d'observation (100) étant notamment formé par :
un nanocanal (110) comprenant une première extrémité (110a) et une deuxième extrémité (110b), et ayant une longueur (L110),
au moins un microcanal (120ab 120b) s'étendant d'un des deux conduits (130a, 130b) d'amenée jusqu'à une extrémité (110a, 100b) du nanocanal, l'autre extrémité du nanocanal étant reliée à l'autre conduit d'amenée,
dans lequel les nanocanaux (110) ont une section (S110) d'aire inférieure à la section (S120) de l'au moins un microcanal (120),
dans lequel, pour au moins deux nanocanaux (110), une dimension parmi leur longueur respective (l110) ou leur section (S110) respective est différente,
et dans lequel la section d'un canal définit une largeur (l), parallèle au plan (XY) et orthogonal à l'axe longitudinal (X), et une profondeur (p), orthogonale au plan (XY) et à l'axe longitudinal (X),
dans lequel les nanocanaux (110) présentent une largeur comprise entre 50 et 500nm, et les microcanaux (120) présentent une largeur comprise entre 1 et 20µm.

2. Dispositif selon la revendication 1, comprenant deux microcanaux, le premier microcanal (120a) s'étendant du premier conduit (130a) d'amenée jusqu'à la première extrémité (110a) du nanocanal, le deuxième microcanal (120b) s'étendant depuis le deuxième conduit d'amenée (130b) jusqu'à la deuxième extrémité (110b) du nanocanal (110).

3. Dispositif selon l'une quelconque des revendications 1 à 2, comprenant au moins trois canaux d'observation (100a, 100b, 100c), dans lequel chaque nanocanal (110) à au moins une desdites dimensions différente du nanocanal (110) des autres canaux d'observation.

4. Dispositif selon l'une quelconque des revendications 1 à 3, dans lequel les microcanaux (120) présentent une largeur comprise entre 1 et 10µm.

5. Dispositif selon l'une quelconque des revendications 1 à 4, dans lequel au moins deux nanocanaux (110), préférablement tous, ont des largeurs (l110) différentes deux à deux.

6. Dispositif selon l'une quelconque des revendications 1 à 5, dans lequel les nanocanaux (110) ont des longueurs (L110) égales.

7. Dispositif selon l'une quelconque des revendications 1 à 5, dans lequel au moins deux nanocanaux (110), préférablement tous les nanocanaux (110), ont des longueurs (L110) différentes deux à deux.

8. Dispositif selon l'une quelconque des revendications 1 à 7, dans lequel les longueurs (Lo) des canaux d'observation (100) sont identiques.

9. Dispositif selon l'une quelconque des revendications 2 à 8, dans lequel les microcanaux (120a, 120b) ont des longueurs différentes.

10. Dispositif selon l'une quelconque des revendications 1 à 9, dans lequel au moins un microcanal (110) a une largeur comprise entre 4 et 6µm et/ou une profondeur comprise entre 0,8 et 1,2 µm.

11. Dispositif selon l'une quelconque des revendications 1 à 10, dans lequel les canaux d'observation (100) s'étendent de façon rectiligne et parallèlement entre eux.

12. Dispositif selon l'une quelconque des revendications 1 à 11, comprenant une plaque (P) définissant le plan XY dans laquelle les canaux d'observation (100) sont gravés sur une face de ladite plaque (P), et comprenant une plaque de capot (C) pour recouvrir les canaux d'observation.

13. Dispositif selon l'une quelconque des revendications 2 à 11, dans lequel, pour au moins un canal d'observation, les deux microcanaux (120a, 120b) dudit canal d'observation (100), ont des largeurs et/ou des longueurs différentes.

14. Système comprenant un dispositif (100) selon l'une quelconque des revendications 1 à 13, et comprenant en outre au moins deux ports (22a, 22b), pour réaliser une communication fluidique avec les canaux d'amenée (130a, 130b), et comprenant des électrodes (30, 32) configurées pour être placées dans les ports (22a, 22b) et une source de tension électrique contrôlable (34) apte à générer une différence de potentiel au sein des canaux d'observation (100) via les électrodes (30, 32).

15. Système selon la revendication 14, comprenant en outre un générateur de pression hydrodynamique contrôlable (40), associé à au moins un des ports (22a, 22b) et apte à générer un gradient de pression au sein des canaux d'observation (100).

16. Système selon l'une quelconque des revendications 14 et 15, comprenant en outre un dispositif d'imagerie (50) configuré pour acquérir des vidéo/images des canaux d'observation (100).

17. Procédé de détection/préconcentration sélective d'analytes chargés contenus dans un électrolyte à l'aide d'un dispositif selon l'une quelconque des revendications 1 à 13 ou à l'aide d'un système selon l'une quelconque des revendications 14 à 16, comprenant les étapes suivantes :
- E1 : injection d'analytes dans le dispositif (100), de sorte que les canaux d'observation (100) soient emplis d'électrolyte,
- E2 : application d'une tension aux extrémités des canaux d'observation (100) pendant une durée déterminée pour provoquer un phénomène d'électropréconcentration et permettre l'apparition de spot de concentrations d'analytes dans l'au moins un microcanal (120).

18. Procédé selon la revendication 17, dans lequel l'étape E2 comprend en outre l'application d'un gradient de pression au sein des canaux d'observation (100) pendant une durée déterminée.

19. Procédé selon l'une quelconque des revendications 17 à 18, dans le cas d'un dispositif selon l'une quelconque des revendications 1 à 13, 19 et 20 comprenant une étape additionnelle E3 de mesure d'au moins une caractéristique suivante sur chacun des microcanaux (120) des différents canaux d'observation (100), pour chaque spot (Sp) :
- position longitudinale dans le microcanal (120) du spot (Sp) de concentration d'analytes,
- taille du spot,
- intensité du spot,
- vitesse de migration du spot,
- largeur du spot.

20. Procédé selon la revendication 19, comprenant une étape additionnelle E4 de comparaison des caractéristiques obtenues à l'étape E3 avec une base de données pour obtenir une information relative à l'analyte.

## Patentansprüche

1. Vorrichtung (100) zur selektiven Erkennung / Vorkonzentration von geladenen Analyten, die in einem Elektrolyt enthalten sind, umfassend:
eine Vielzahl von Beobachtungskanälen (100a, 100b, ...), die sich jeweils gemäß einer Längsrichtung (X) in einer Ebene (XY) erstrecken,
zwei Zuführkanäle (130a, 130b), die miteinander durch die Vielzahl von Beobachtungskanälen (100) verbunden und ausgestaltet sind, um das Elektrolyt in die genannte Vielzahl von Beobachtungskanälen (100) zu verbringen,
wobei jeder Beobachtungskanal (100) insbesondere gebildet ist durch:
einen Nanokanal (110), umfassend ein erstes Ende (110a) und ein zweites Ende (110b) und eine Länge (L110) aufweist,
wenigstens einen Mikrokanal (120ab, 120b), der sich von einer der zwei Zuführleitungen (130a, 130b) bis zu einem Ende (110a, 110b) des Nanokanals erstreckt, wobei das andere Ende des Nanokanals mit der anderen Zuführleitung verbunden ist,
bei der die Nanokanäle (110) einen Bereichsquerschnitt (S110) aufweisen, der kleiner ist als der Querschnitt (S120) wenigstens eines Mikrokanals (120),
bei der bei wenigstens einem der zwei Mikrokanäle (110) eine Abmessung aus ihrer jeweiligen Länge (l110) oder ihrem jeweiligen Querschnitt (S110) unterschiedlich ist,
und bei der der Querschnitt eines Kanals eine Breite (1), die parallel zur Ebene (XY) und orthogonal zur Längsachse (X) ist, und eine Tiefe (p), die orthogonal zur Ebene (XY) und zur Längsachse (X) ist, definiert,
bei der die Nanokanäle (110) eine zwischen 50 und 500 nm inbegriffene Breite aufweisen und die Mikrokanäle (120) eine zwischen 1 und 20 µm inbegriffene Breite aufweisen.

2. Vorrichtung gemäß Anspruch 1, umfassend zwei Mikrokanäle, wobei sich der erste Mikrokanal (102a) von der ersten Zuführleitung (130a) bis zum ersten Ende (110a) des Nanokanals erstreckt, wobei sich der zweite Mikrokanal (120b) von der zweiten Zuführleitung (130b) bis zum zweiten Ende (110b) des Nanokanals (110) erstreckt.

3. Vorrichtung gemäß irgendeinem der Ansprüche 1 bis 2, umfassend wenigstens drei Beobachtungskanäle (100a, 100b, 100c), bei denen jeder Nanokanal (110) wenigstens eine der genannten Abmessungen aufweist, die von dem Nanokanal (110) der anderen Beobachtungskanäle unterschiedlich ist.

4. Vorrichtung gemäß irgendeinem der Ansprüche 1 bis 3, bei der die Mikrokanäle (120) eine zwischen 1 und 10 µm inbegriffene Breite aufweisen.

5. Vorrichtung gemäß irgendeinem der Ansprüche 1 bis 4, bei der wenigstens zwei Nanokanäle (110), bevorzugt alle, paarweise unterschiedliche Breiten (l110) aufweisen.

6. Vorrichtung gemäß irgendeinem der Ansprüche 1 bis 5, bei der die Nanokanäle (110) gleichen Längen (L110) aufweisen.

7. Vorrichtung gemäß irgendeinem der Ansprüche 1 bis 5, bei der wenigstens zwei Nanokanäle (110), bevorzugt alle Nanokanäle (110), paarweise unterschiedliche Längen (L110) aufweisen.

8. Vorrichtung gemäß irgendeinem der Ansprüche 1 bis 7, bei der die Längen (Lo) der Beobachtungskanäle (100) identisch sind.

9. Vorrichtung gemäß irgendeinem der Ansprüche 2 bis 8, bei der die Mikrokanäle (120a, 120b) unterschiedliche Längen aufweisen.

10. Vorrichtung gemäß irgendeinem der Ansprüche 1 bis 9, bei der wenigstens ein Mikrokanal (110a) eine zwischen 4 und 6 µm inbegriffene Breite und / oder eine zwischen 0,8 und 1,2 µm inbegriffene Tiefe aufweist.

11. Vorrichtung gemäß irgendeinem der Ansprüche 1 bis 10, bei der die Beobachtungskanäle (100) sich geradlinig und zueinander parallel erstrecken.

12. Vorrichtung gemäß irgendeinem der Ansprüche 1 bis 11, umfassend eine Platte (P), die die Ebene XY definiert, in der die Beobachtungskanäle (100) auf einer Seite der genannten Platte (P) geätzt sind, und umfassend eine Abdeckplatte (C) zum Abdecken der Beobachtungskanäle.

13. Vorrichtung gemäß irgendeinem der Ansprüche 2 bis 11, bei der für wenigstens einen Beobachtungskanal die zwei Mikrokanäle (120a, 120b) des genannten Beobachtungskanals (100) unterschiedliche Breiten und / oder Längen aufweisen.

14. System, umfassend eine Vorrichtung (100) gemäß irgendeinem der Ansprüche 1 bis 13 und umfassend darüber hinaus wenigstens zwei Ports (22a, 22b) zum Realisieren einer fluidischen Verbindung mit den Zuführkanälen (130a, 130b) und umfassend Elektroden (30, 32), die ausgestaltet sind, um in den Ports (22a, 22b) platziert zu sein, und eine steuerbare, elektrische Spannungsquelle (34), die geeignet ist, eine Potenzialdifferenz innerhalb der Beobachtungskanäle (100) über die Elektroden (30, 32) zu erzeugen.

15. System gemäß Anspruch 14, umfassend darüber hinaus einen steuerbaren hydrodynamischen Druckgenerator (40), der wenigstens einem der Ports (22a, 22b) zugeordnet ist und geeignet ist, einen Druckgradienten innerhalb der Beobachtungskanäle (100) zu erzeugen.

16. System gemäß irgendeinem der Ansprüche 14 und 15, umfassend darüber hinaus eine Bildgebungsvorrichtung (50), die ausgestaltet ist, um Videos / Bilder der Beobachtungskanäle (100) zu erwerben.

17. Verfahren zur selektiven Erkennung / Vorkonzentration von geladenen Analyten, die in einem Elektrolyt enthalten sind, mithilfe einer Vorrichtung gemäß irgendeinem der Ansprüche 1 bis 13 oder mithilfe eines Systems gemäß irgendeinem der Ansprüche 14 bis 16, umfassend die folgenden Schritte:
- E1: Einschießen von Analyten in die Vorrichtung (100) derart, dass die Beobachtungskanäle (100) mit Elektrolyt gefüllt sind,
- E2: Anwenden einer Spannung auf die Enden der Beobachtungskanäle (100) während einer bestimmten Dauer, um ein Elektrovorkonzentrations-Phänomen hervorzurufen und das Auftreten von Konzentrationsspots von Analyten in dem wenigstens einen Mikrokanal (120) zu ermöglichen.

18. Verfahren gemäß Anspruch 17, bei dem der Schritt E2 darüber hinaus während einer bestimmten Dauer die Anwendung eines Druckgradienten innerhalb der Beobachtungskanäle (100) umfasst.

19. Verfahren gemäß irgendeinem der Ansprüche 17 bis 18 in dem Fall einer Vorrichtung gemäß irgendeinem der Ansprüche 1 bis 13, 19 und 20, umfassend einen zusätzlichen Schritt E3 zum Messen wenigstens eines folgenden Merkmals auf jedem der Mikrokanäle (120) der unterschiedlichen Beobachtungskanäle (100) für jeden Spot (Sp):
- Längsposition in dem Mikrokanal (120) des Spots (Sp) der Analytenkonzentration,
- Größe des Spots,
- Intensität des Spots,
- Migrationsgeschwindigkeit des Spots,
- Breite des Spots.

20. Verfahren gemäß Anspruch 19, umfassend einen zusätzlichen Schritt E4 zum Vergleichen der in Schritt E3 erhaltenen Merkmale mit einer Datenbank zum Erhalten einer Information bezüglich des Analyts.

## Claims

1. Device (100) for the selective detecting/preconcentrating of charged analytes contained in an electrolyte, comprising:
a plurality of observation channels (100a, 100b, ...) each extending in a longitudinal direction (X) in a plane (XY),
two feed channels (130a, 130b) connected together by the plurality of observation channels (100) and configured to feed the electrolyte into said plurality of observation channels (100),
each observation channel (100) being in particular formed by:
a nanochannel (110) comprising a first end (110a) and a second end (110b), and having a length (L110),
at least one microchannel (120ab 120b) extending from one of the two feed lines (130a, 130b) to one end (110a, 100b) of the nanochannel, the other end of the nanochannel being connected to the other feed line,
wherein the nanochannels (110) have a section (5110) of area less than the section (S120) of the at least one microchannel (120),
wherein, for at least two nanochannels (110), a dimension among the respective length (1110) thereof or the respective section (S110) thereof is different,
and wherein the section of a channel defines a width (1), parallel to the plane (XY) and orthogonal to the axis longitudinal (X), and a depth (p), orthogonal to the plane (XY) and to the axis longitudinal (X),
wherein the nanochannels (110) have a width comprised between 50 and 500nm, and the microchannels (120) have a width comprised between 1 and 20µm.

2. Device according to claim 1, comprising two microchannels, the first microchannel (120a) extending from the first feed line (130a) to the first end (110a) of the nanochannel, the second microchannel (120b) extending from the second feed line (130b) to the second end (110b) of the nanochannel (110).

3. Device according to any of claims 1 to 2, comprising at least three observation channels (100a, 100b, 100c), wherein each nanochannel (110) has a least one of said dimensions different from the nanochannel (110) of the other observation channels.

4. Device according to any of claims 1 to 3, wherein the microchannels (120) have a width comprised between 1 and 10µm.

5. Device according to any of claims 1 to 4, wherein at least two nanochannels (110), preferably all of them, have different widths (1110) two by two.

6. Device according to any of claims 1 to 5, wherein the nanochannels (110) have equal lengths (L110) .

7. Device according to any of claims 1 to 5, wherein at least two nanochannels (110), preferably all the nanochannels (110), have different lengths (L110) two by two.

8. Device according to any of claims 1 to 7, wherein the lengths (Lo) of the observation channels (100) are identical.

9. Device according to any of claims 2 to 8, wherein the microchannels (120a, 120b) have different lengths.

10. Device according to any of claims 1 to 9, wherein at least one microchannel (110) has a width comprised between 4 and 6µm and/or a depth comprised between 0.8 and 1.2 µm.

11. Device according to any of claims 1 to 10, wherein the observation channels (100) extend strait and parallel between them.

12. Device according to any of claims 1 to 11, comprising a plate (P) defining the plane XY wherein the observation channels (100) are etched on a face of said plate (P), and comprising a cover plate (C) to cover the observation channels.

13. Device according to any of claims 2 to 11, wherein, for at least one observation channel, the two microchannels (120a, 120b) of said observation channel (100), have different widths and/or lengths.

14. System comprising a device (100) according to any of claims 1 to 13, and further comprising at least two ports (22a, 22b), in order to carry out a fluid communication with the feed channels (130a, 130b), and comprising electrodes (30, 32) configured to be placed in the ports (22a, 22b) and a controllable source of electrical voltage (34) able to generate a difference in potential within the observation channels (100) via the electrodes (30, 32).

15. System according to claim 14, further comprising a controllable hydrodynamic pressure generator (40), associated with at least one of the ports (22a, 22b) and able to generate a pressure gradient within the observation channels (100).

16. System according to any of claims 14 and 15, further comprising an imaging device (50) configured to acquire video/images of the observation channels (100).

17. Method for the selective detecting/preconcentrating of charged analytes contained in an electrolyte using a device according to any of claims 1 to 13 or using a system according to any of claims 14 to 16, comprising the following steps:
- E1: injecting of analytes into the device (100), in such a way that the observation channels (100) are filled with electrolyte,
- E2: applying a voltage to the ends of the observation channels (100) for a determined duration in order to provoke an electropreconcentration phenomenon and allow for the appearance of spot of analyte concentrations in at least one microchannel (120).

18. Method according to claim 17, wherein step E2 further comprises the application of a pressure gradient within the observation channels (100) for a determined duration.

19. Method according to any of claims 17 to 18, in the case of a device according to any of claims 1 to 13, 19 and 20 comprising an additional step E3 of measuring at least one following characteristic on each one of the microchannels (120) of the various observation channels (100), for each spot (Sp):
- longitudinal position in the microchannel (120) of the analyte concentration spot (Sp),
- size of the spot,
- intensity of the spot,
- migration speed of the spot,
- width of the spot.

20. Method according to claim 19, comprising an additional step E4 of comparing characteristics obtained in step E3 with a database in order to obtain information concerning the analyte.
